# EUROPEAN PATENT APPLICATION

(11) **EP 2 258 438 A1**
(43) Date of publication of application: **08.12.2010**
(21) Application number: 10178777.8
(22) Date of filing: 08.08.2002
(51) Int. Cl.: A61M 25/01

(54) **Roller wheel assisted guidewire advancer**

(30) Priority: 15.08.2001 US 930509
(62) Divisional of application: 02765958.0
(71) Applicant: Becton Dickinson and Company, Franklin Lakes, New Jersey 07417-1880 (US)
(72) Inventor: Heh, Kok Boon, 768427, Singapore (SG); Widjaja, Anton, 730703, Singapore (SG)
(74) Representative: Williams, Gareth Owen

(57) **Abstract**

The invention relates to a guidewire advancer having a roller wheel to facilitate the advancement of the guidewire from the guidewire advancer and into a patient's blood vessel. If desired the roller wheel can have a roughened or slotted circumferential surface.

## Description

### Background of the Invention

The subject invention relates to a device that allows the controlled insertion of a guidewire into a blood vessel. Such a device is referred to generally as a guidewire advancer. More particularly, the subject invention relates to a guidewire advancer having a roller wheel to facilitate the advancement of the guidewire from the guidewire advancer and into a patient's blood vessel.

Guidewires are well known in the art. They are typically made from a tightly wound surgical grade stainless steel wire of small diameter so as to be highly flexible yet stiff and resilient. These characteristics allow guidewires to be inserted into a patient's vasculature or body cavity and accurately positioned therein. Although guidewires may be employed in a number of different ways, they are generally used to introduce a catheter into a blood vessel.

For example, catheters such as midline catheters, central catheters and peripherally inserted central catheters may be used for certain types of IV therapy. Specifically these catheters are used when certain harsh medicaments must be infused into the patient and must be diluted quickly in a large vein to prevent adverse reaction by the patient. In such a situation, it is important for the distal tip of the catheter to be located in the auxiliary, subclavian or brachiocephalic vein or in the superior vena cava. Midline catheters, central catheters and peripherally inserted central catheters, which can be on the order of several inches long or longer, fill this need.

For these longer catheters, guidewires can be used to facilitate the introduction and proper placement of the catheter into a patient. One method of inserting guidewires and catheters into a patient is by the modified Seldinger technique. With this technique, the clinician first inserts a hypodermic needle via a syringe into the patient's blood vessel. Vascular access is confirmed by pulling a small amount of blood through the hypodermic needle into the syringe.
Thereafter, the syringe is removed leaving the hypodermic needle in place. The clinician then inserts a guidewire through the hypodermic needle into the patient and advances the guidewire until the distal end of the guidewire reaches the target site. Thereafter, the catheter is inserted into the patient over the guidewire, which acts as a track to facilitate the proper placement of the catheter in the patient. Alternatively, an introducer can be inserted into the patient's vasculature instead of the hypodermic needle and the guidewire can be advanced into the patient through the introducer.

Because of the tortuous path of a patient's vasculature, a long guidewire is needed to ensure that the distal end of the guidewire is adjacent to the target site and a suitable length of the guidewire extends outside of the patient's body. A length of guidewire must extend outside of the patient's body to allow the clinician to be able to handle the guidewire and thread the catheter onto the proximal end of the guidewire without losing control of the guidewire placement within the patient's body. Thus, guidewires can be many feet in length. However, such a length makes the initial insertion of the guidewire into a patient awkward and difficult. Previously, an assistant would control the proximal portion of the guidewire as the clinician inserted the guidewire into the patient. In these cost conscious times, the employment of an assistant during guidewire placement is unacceptable.

Devices currently on the market have been developed to avoid the need for an assistant during guidewire placement. Such devices known as guidewire advancers include a hollow coiled plastic tube that houses the guidewire and makes it easier for the clinician to handle the guidewire. In addition, the tube may include an aperture adjacent to one end of the tube to allow the clinician to access the guidewire. In this way, the clinician could place a finger through the aperture, contact the guidewire and, with the friction between the clinician's finger and the guidewire, advance the guidewire from the tube and into the patient.

Although these devices work generally for their intended purpose they could be improved. For example, during such medical procedures, the clinician's hands will become covered with blood and other body fluids making the clinician's hands slippery. In addition, the guidewire and the guidewire advancer will become covered with blood and other body fluids. This will make it difficult for the clinician to properly grip the guidewire to advance it from the guidewire advancer into the patient's blood vessel. In addition, because the guidewire will feel slippery, it will be difficult for the clinician to accurately gauge how much of the guidewire has actually been advanced from the guidewire advancer into the patient. In addition, these previous devices may have a flat surface under the aperture against which the clinician manipulates the guidewire. Such a flat surface exacerbates the problem of poor frictional contact between the clinician's finger and the guidewire and gives the clinician a poorer feel of the guidewire as discussed above. Previous designs also could be improved ergonomically.

### Summary of the Invention

It is therefore an object of this invention to provide a guidewire advancer that allows the clinician to securely grip the guidewire so that the guidewire can be easily advanced from the guidewire advancer and into a patient.

It is another object of this invention to provide a guidewire advancer that allows the clinician to accurately gauge how much of the guidewire has been advanced from the guidewire advancer into the patient.

It is still another object of this invention to provide a guidewire advancer that has an improved ergonomic design to provide the clinician with a proper feel of the guidewire.

The guidewire advancer of this invention comprises an elongate tube having a first outlet at a first end and a second outlet at a second end, a body member having a proximal portion, a central portion and a distal portion, wherein the proximal portion of the body member is connected to the first outlet of the elongate tube, and a roller wheel rotatably connected to the body member adjacent to the central portion thereof. Both the proximal portion and the distal portion of the body member define a passage therein for allowing a guidewire to extend therethrough. Preferably, the passage of the proximal portion of the body member is radially offset from the passage of the distal portion of the body member such that the passage of the proximal portion of the body member is above the passage of the distal portion of the body member.

The roller wheel defines a circumferential surface that is rough and is preferably serrated where the peaks are radiused and extend across the width of the circumferential surface. Alternatively, the circumferential surface could be formed with some other configuration that results in a rough surface having a high coefficient of friction as compared to a smooth surface. For example, the circumferential surface could be formed with a plurality of protrusions or recesses, such as pits or cross hatched score lines formed therein. In addition, the roller wheel could be formed such that the circumferential surface is formed as a channel or slot with sidewalls. The axis about which the roller wheel rotates is preferably radially offset from the passage of the proximal portion of the body member such that the axis is below the passage of the proximal portion of the body member. Even more preferably, the circumferential surface of the roller wheel is below the passage of the proximal portion of the body member.

If desired, an end cap may be placed over the second outlet of the elongate tube to prevent the guidewire from slipping out the back of the guidewire advancer.

### Brief Description of the Drawings

The preferred embodiments are illustrated in the drawings in which like reference numerals refer to like elements and in which:

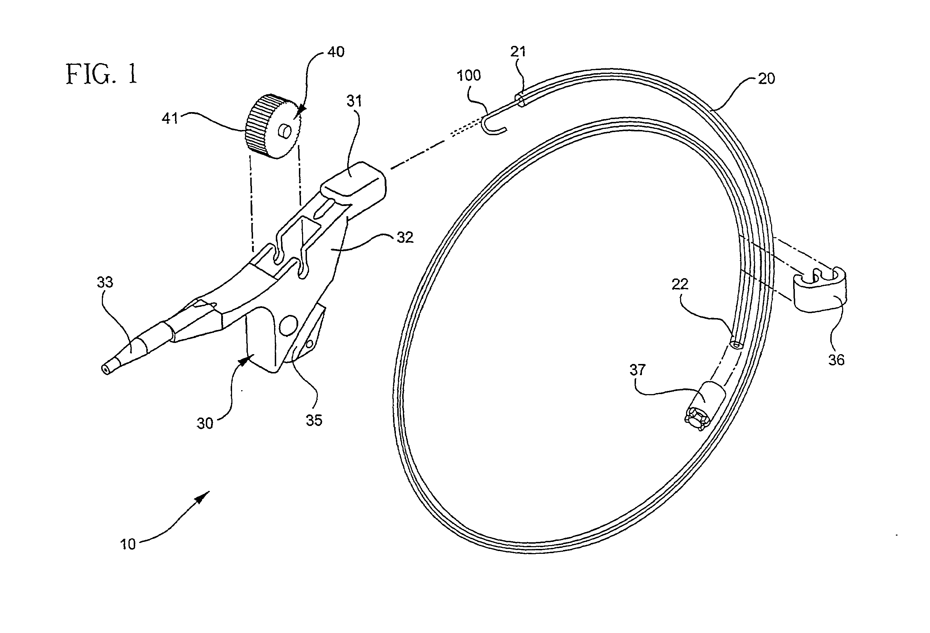

FIG. 1 is an exploded perspective view of the roller wheel assisted guidewire advancer of this invention;

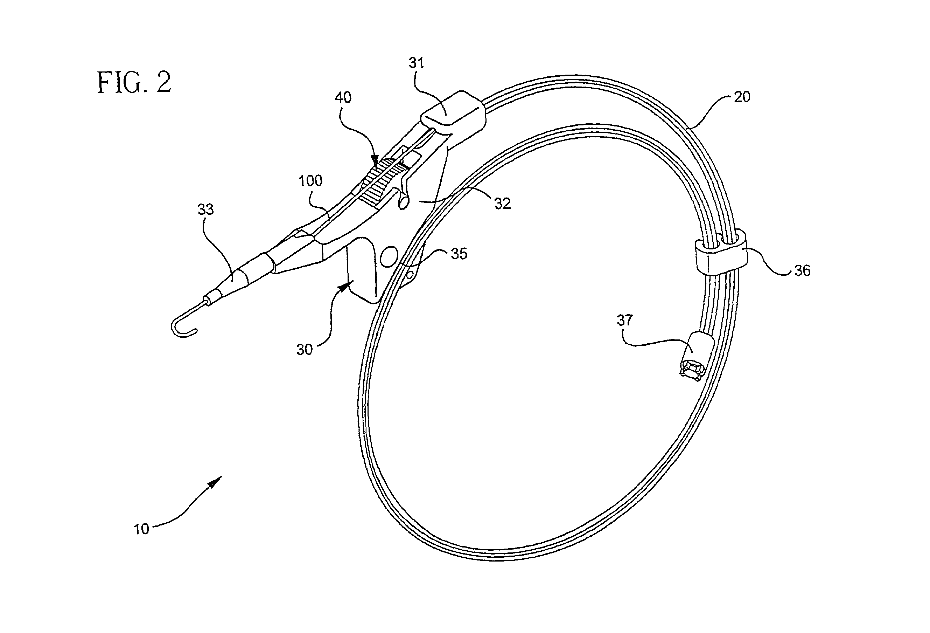

FIG. 2 is a perspective view of the roller wheel assisted guidewire advancer of this invention;

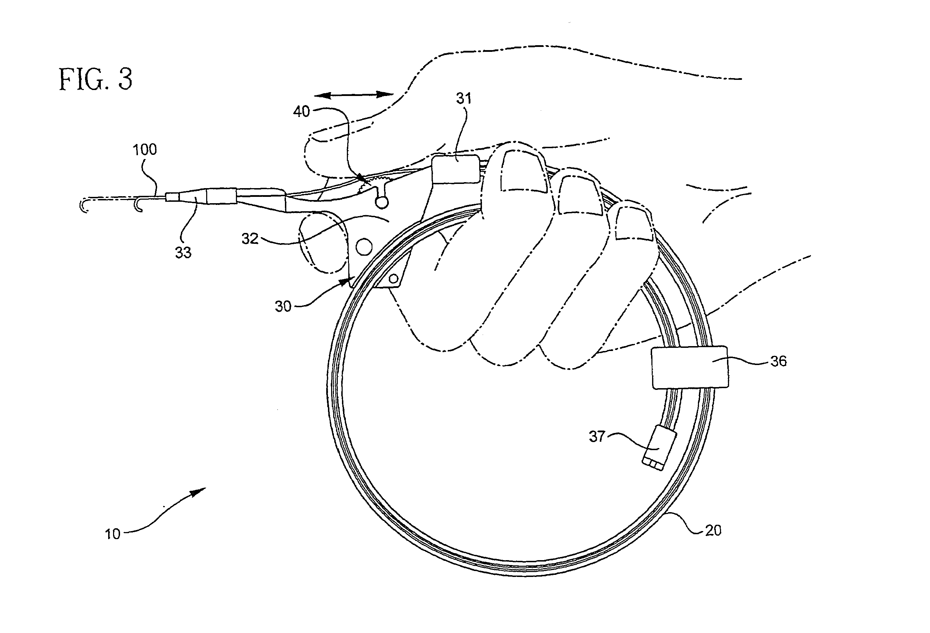

FIG. 3 is a side elevation view of the roller wheel assisted guidewire advancer of this invention showing a clinician's hand in phantom to demonstrate how the roller assisted guidewire advancer of this invention is used;
FIG. 4 is an enlarged cross sectional view of the body member of the roller wheel assisted guidewire advancer of this invention incorporating the roller wheel;
FIG. 5 is an enlarged perspective view of one embodiment of the roller wheel used in the roller wheel assisted guidewire advancer of this invention;
FIG. 6 is an enlarged perspective view of a second embodiment of the roller wheel used in the roller wheel assisted guidewire advancer of this invention;
FIG. 7 is an enlarged perspective view of a third embodiment of the roller wheel used in the roller wheel assisted guidewire advancer of this invention;
FIG. 8 is an enlarged perspective view of a fourth embodiment of the roller wheel used in the roller wheel assisted guidewire advancer of this invention; and
FIG. 9 is an enlarged perspective view of a fifth embodiment of the roller wheel used in the roller wheel assisted guidewire advancer of this invention.

### Detailed Description of the Invention

As used herein, the term "proximal" refers to a location on the device that is closest to the clinician using the device and farthest from the patient in connection with whom the device is used when the device is used in its normal operation. Conversely, the term "distal" refers to a location on the device that is farthest from the clinician using the device and closest to the patient in connection with whom the device is used when the device is used in its normal operation.

As used herein, the term "top", "up", "above" or "upwardly" refers to a location with respect to the device that, during normal use, is radially away from the longitudinal axis of the device and away from the patient's skin. Conversely, as used herein, the term "bottom", down", "below" or "downwardly" refers to a location with respect to the device that, during normal use, is radially away from the longitudinal axis of the device and toward the patient's skin.

As used herein, the term "in" or "inwardly" refers to a location with respect to the device that, during normal use, is toward the inside of the device. Conversely, as used herein, the term "out" or "outwardly" refers to a location with respect to the device that, during normal use, is toward the outside of the device.

The guidewire advancer 10 of this invention comprises an elongate tube 20 having a first outlet 21 at its distal end and a second outlet 22 at its proximal end, a body member 30 having a proximal portion 31, a central portion 32 and a distal portion 33. First outlet 21 of elongate tube 20 is connected to proximal portion 31. Tube 20 is preferably coiled to facilitate handling of guidewire advancer 10 by a clinician. If desired, a slot 35 may be formed in body member 30 to allow tube 20 to fit therein and hold tube 20 in a coiled position. In addition, a clip 36 may be used to clip adjacent sections of tube 20 together to hold tube 20 in a coiled position. A roller wheel 40 is rotatably connected to body member 30 adjacent to central portion 32 so that the axis of rotation of roller wheel is transverse to the longitudinal axis of body member 30. Proximal portion 31 of body member 30 defines a proximal passage 31 a therein to allow a guidewire 100 to extend therethrough. Similarly, distal portion 33 of body member 30 defines a distal passage 33a therein to allow guidewire 100 to extend therethrough. When guidewire 100 is located in guidewire advancer 10, guidewire 100 extends through tube 20, proximal passage 31 a, over roller wheel 40 and through distal passage 33a. This configuration allows the clinician to easily access guidewire 100 adjacent to body member 30 in the space between proximal passage 31 a and distal passage 33a. Preferably, the clinician's finger contacts guidewire 100 when guidewire 100 is on roller wheel 40. In this way, the clinician can securely grip guidewire 100 against roller wheel 40 and thus can easily advance guidewire 100 as roller wheel 40 is rotated in the direction of the desired movement of guidewire 100. In addition, the clinician can accurately gauge how much of guidewire 100 has been advanced from guidewire advancer 10 into the patient because the clinician will be able to feel or see roller wheel 40 rotate as guidewire 100 is advanced from guidewire advancer 10.

Preferably, proximal passage 31 a is radially offset from distal passage 33a such that proximal passage 31 a is above distal passage 33a. This radially offset arrangement creates a tortuous path for guidewire 100 as it extends between proximal passage 31 a and distal passage 33a. Proximal passage 31 a and distal passage 33a thus gently hold guidewire 100 in place and significantly reduce the tendency for guidewire 100 to drift out of guidewire advancer 10 when it is being shipped, handled or otherwise manipulated. This radially offset arrangement also presents an ergonomically beneficial arrangement for the clinician. Although it is possible for proximal passage 31 a to be below or axially aligned with distal passage 33a, such an arrangement is not preferred because it will not supply all of the advantages achieved when proximal passage 31 a is above distal passage 33a.

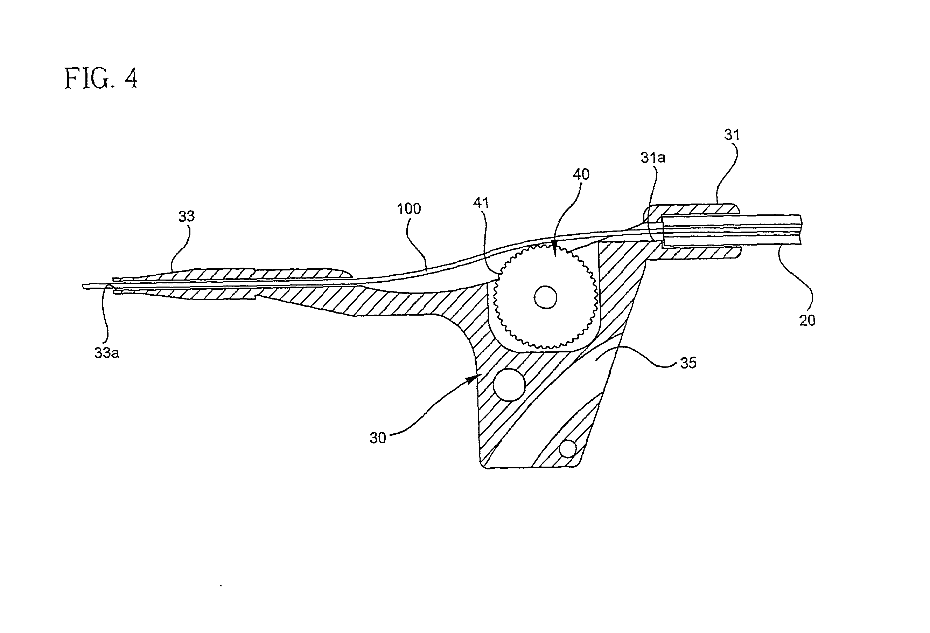

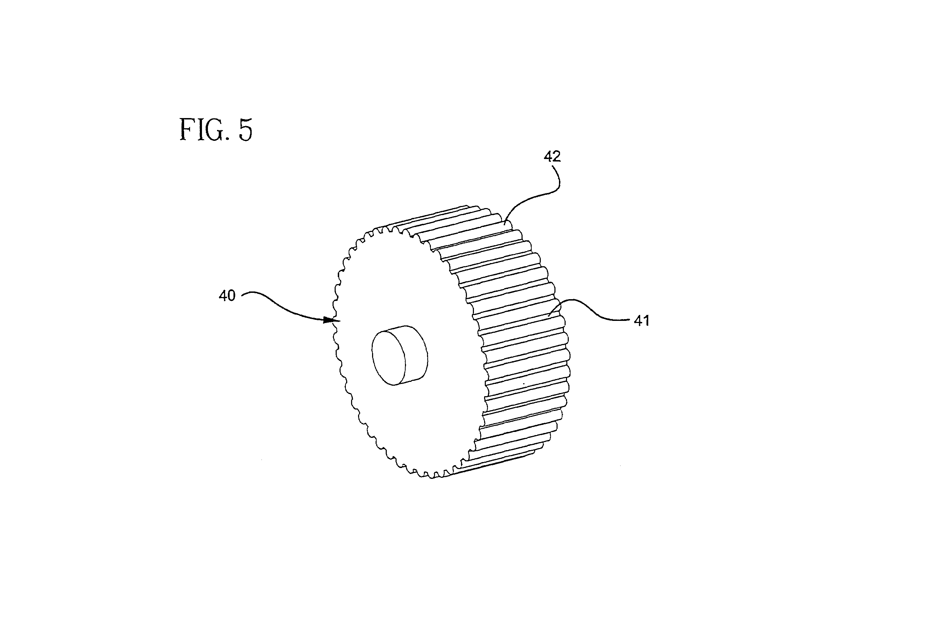

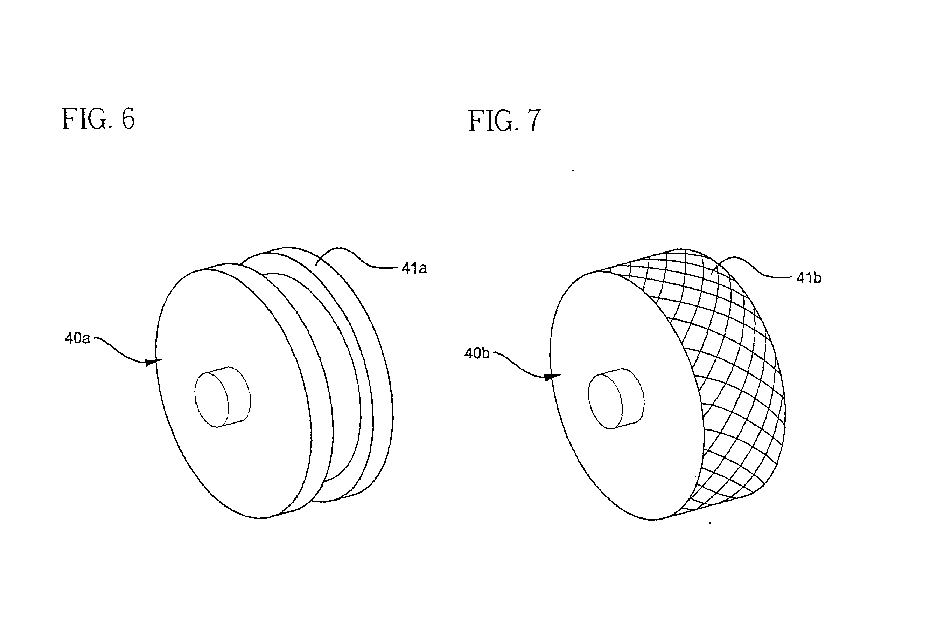

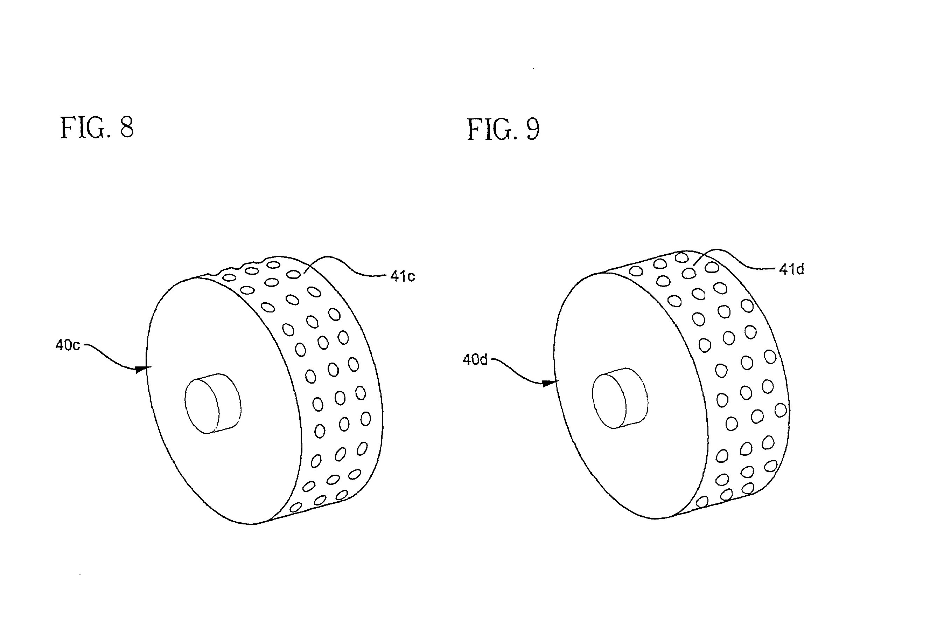

Roller wheel 40 defines a circumferential surface 41 that is rough and preferably is serrated where the peaks 42 are radiused and the serrations extend across the width of circumferential surface 41, i.e. parallel to the axis of rotation of roller wheel 40. See FIGS. 4 and 5. Alternatively, the circumferential surface could be formed with some other configuration that results in a rough surface having a high coefficient of friction as compared to a smooth surface. For example, the circumferential surface could be formed with a plurality of protrusions or recesses, such as pits or cross hatched score lines formed therein. See circumferenatial surfaces 41 b - 41 c in FIGS. 7 - 9. In addition, the circumferential surface could include a rubberized surface or otherwise be covered with a material having a high coefficient of friction as compared to a smooth surface.

Roller wheel 40 should be wide enough to minimize the chances that guidewire 100 will slide off of the side of circumferential surface 41 during use. For example, roller wheel 40 should be at least about 3 millimeters wide when used with a standard guidewire. Alternatively, roller wheel 40 could be formed such that the circumferential surface is formed as a channel or slot with sidewalls to affirmatively hold guidewire 100 therein. See circumferential surface 41 a in FIG. 6. Such a configuration ensures that guidewire 100 will not become disengaged from the surface of roller wheel 40.

The axis about which roller wheel 40 rotates is preferably radially offset from proximal passage 31 a such that the axis is below distal passage 33a. Preferably this results in the top of roller wheel 40 being slightly above distal passage 33a such that it is aligned with or slightly above a line connecting the distal end of proximal passage 31 a with the proximal end of distal passage 33a. With this arrangement, and where proximal passage 31 a is above distal passage 33a, circumferential surface 41 will extend into the path of guidewire 100 as it extends between proximal passage 31 a and distal passage 33a. This will ensure that circumferential surface 41 will frictionally engage guidewire 100 to prevent unwanted movement of guidewire 100 with respect to guidewire advancer 10 and will allow the clinician to easily grip and advance guidewire 100.

If desired, an end cap 37 may be placed over the second outlet of elongate tube 20 to prevent guidewire 100 from slipping out the back of guidewire advancer 10.

Thus, it is seen that a guidewire advancer is provided that allows the clinician to securely grip the guidewire so that the guidewire can be easily advanced from the guidewire advancer and into a patient, to accurately gauge how much of the guidewire has been advanced from the guidewire advancer into the patient and that has an improved ergonomic design to provide the clinician with a proper feel of the guidewire.

## Claims

1. A guidewire advancer, comprising: an elongate tube having a first outlet at a first end and a second outlet at a second end; a body member having a proximal portion, a central portion and a distal portion, wherein the proximal portion is connected to the first outlet of the elongate tube; and a roller wheel rotatably connected to the body member adjacent to the central portion thereof.

2. The guidewire advancer of claim 1 wherein the proximal portion of the body member defines a passage therein for allowing a guidewire to extend therethrough.

3. The guidewire advancer of claim 1 wherein the distal portion of the body member defines a passage therein for allowing a guidewire to extend therethrough.

4. The guidewire advancer of claim 1 wherein the passage of the proximal portion of the body member is radially offset from the passage of the distal portion of the body member.

5. The guidewire advancer of claim 4 wherein the passage of the proximal portion of the body member is above the passage of the distal portion of the body member.

6. The guidewire advancer of claim 1 wherein the roller wheel defines a circumferential surface that is rough.

7. The guidewire advancer of claim 1 wherein the roller wheel defines a circumferential surface that is rubberized.

8. The guidewire advancer of claim 1 wherein the circumferential surface of the roller wheel defines a channel therein.

9. The guidewire advancer of claim 1 wherein the roller wheel defines an axis about which the roller wheel rotates and wherein the axis of the roller wheel is radially offset from the passage of the proximal portion of the body member.

10. The guidewire advancer of claim 9 wherein the axis of the roller wheel is below the passage of the proximal portion of the body member.

11. The guidewire advancer of claim 10 wherein the circumferential surface of the roller wheel is above the passage of the distal portion of the body member.

12. The guidewire advancer of claim 1 further comprising an end cap covering the second outlet of the elongate tube.

13. The guidewire advancer of any preceding claim further comprising a guidewire disposed in the elongate tube and extending through a passage in the proximal portion of the body member, over the roller wheel and through a passage in the distal portion of the body member.

14. The guidewire advancer of claim 1 wherein the proximal portion defines a passage therein; and the distal portion defines a passage therein; the advancer further comprising a guidewire disposed in the elongate tube and extending through the passage in the proximal portion of the body member, over the roller wheel and through the passage in the distal portion of the body member.

15. The guidewire advancer of claim 14 wherein the roller wheel has a circumferential surface defining a channel therein, or has a rough circumferential surface, or defines a circumferential surface that is rubberised.
